# EUROPEAN PATENT APPLICATION

(11) **EP 1 088 826 A1**
(43) Date of publication of application: **04.04.2001**
(21) Application number: 00127571.8
(22) Date of filing: 30.07.1996
(51) Int. Cl.: C07D 503/00

(54) **Process for the preparation of potassium clavulanate**

(30) Priority: 02.08.1995 GB 9515809
(62) Divisional of application: 96927643.5
(71) Applicant: SMITHKLINE BEECHAM PLC, Brentford, Middlesex TW8 9EP (GB)
(72) Inventor: Cook, Michael Allen, Worthing, West Sussex BN14 8QH (GB)
(74) Representative: Walker, Ralph Francis, Dr.

(57) **Abstract**

A process for preparation of potassium clavulanate by the direct precipitation of clavulanic acid as the potassium salt by providing a solution of clavulanic acid in an organic solvent and mixing a potassium salt with this solution, with between 0.1 % v:v and 7.5 % v:v of water present in the region where the clavulanic acid and potassium salt contact, then isolating the so-formed potassium clavulanate.

## Description

This invention relates to a novel process for the preparation of salts of clavulanic acid.

Clavulanic acid (I): 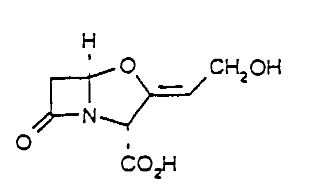 is a β-lactamase inhibitor which is used commercially as a component of pharmaceutical formulations, usually in the form of its salts. Clavulanic acid is produced commercially by culture of the microorganism *Streptomyces clavuligerus,* for example as described in GB 1508977.

Clavulanic acid or its salts may be extracted from the culture medium in various ways but one way this is achieved is that the cells of the *S. clavuligerus* are first removed from the culture medium by such methods as filtration or centrifugation before such extraction procedures are commenced.

Clavulanic acid or its salts may be extracted from clarified culture medium by a variety of methods. Solvent extraction from cold clarified culture medium adjusted to acid pH values and methods which utilize the anionic nature of clavulanic acid at neutral pH such as the use of anion exchange resins have been found to be particularly useful. A further particularly useful method is to form an ester of clavulanic acid, purify the ester and regenerate the acid or its salt therefrom.

The extraction processes for obtaining clavulanic acid or its salts may notionally be divided into a primary isolation process followed by a further purification process.

Suitable primary isolation processes include solvent extraction of the free clavulanic acid. In the solvent extraction process the clavulanic acid is extracted into an organic solvent from cold clarified culture medium, which may be whole broth, adjusted to an acid pH value.

In one solvent extraction process for clavulanic free acid the clarified medium is chilled and the pH lowered into the region of pH 1-2 by the addition of acid while mixing with a substantially water-imiscible organic solvent. Suitable acids used to lower the pH include hydrochloric, sulphuric, nitric, phosphoric or the like mineral acids. Suitable organic solvents include n-butanol, ethyl acetate, n-butyl acetate and methyl isobutyl ketone, and other similar solvents. Methyl isobutyl ketone is a particularly suitable solvent for use in the extraction of the acidified culture filtrate. After separation of the phases clavulanic acid is found in solution in the organic phase.

The clavulanic acid may be back extracted from the organic phase into a new aqueous phase by making use of the greater water solubility of, for example, the alkali metal or alkaline earth metal salts of clavulanic acid in water than in organic solvents. Thus the clavulanic acid may be back extracted from the organic solvent into an aqueous solution or suspension of an alkali metal or alkaline earth metal base, such as sodium hydrogen carbonate, potassium hydrogen phosphate buffer or calcium carbonate, or water, while maintaining the pH at approximately neutrality, for example pH 7. This aqueous extract, after separation of the phases, may be concentrated under reduced pressure. Freeze-drying may also be employed to provide a solid crude preparation of the salt of clavulanic acid. Such solid preparations are stable when stored as a dry solid at -20°C. A similar process is described in GB 1563103. This process may be modified in known ways by for example additional purification steps applied to the organic solvent phase to remove high molecular weight impurities from the impure clavulanic acid.

A further secondary purification process for clavulanic acid is that described in for example EP 0026044, in which a solution of impure clavulanic acid in an organic solvent is contacted with t-butylamine to form the t-butylamine salt of clavulanic acid, which is then isolated, thereby separating the clavulanic acid from impurities remaining in the organic solvent, and the salt is then converted back to clavulanic acid or into a derivative of clavulanic acid such as an alkali metal salt or an ester. Other known secondary purification processes for clavulanic acid involve the use of other organic amines such as diethylamine, tri-(lower alkyl) amines, dimethylaniline and NN'-diisopropylethylenediamine to form salts and/or other derivatives thereof with the clavulanic acid. These purification process have the inherent disadvantage that they can introduce traces of the amine, or leave residual traces of salts of clavulanic acid with the amine, in the final product.

Such back extraction processes present a problem when potassium clavulanate is prepared, as potassium clavulanate is particularly water-sensitive. In conventional back extraction processes potassium clavulanate can remain in contact with water for a long time, typically around an hour or more as the solution concentration of potassium clavulanate builds up under the relatively gentle mixing and separating conditions generally used, and this can lead to extensive hydrolytic degradation. Furthermore such a process involving the participation of clavulanate acid via certain amine salts as described above results in a relatively expensive process.

WO 96/28452 discloses a process in which a dry solution of clavulanic acid in an organic solvent is mixed with a metal donor and at least one additional solvent, to form a metal clavulanate salt.

The inventors have discovered an improved process for the preparation of salts of clavulanic acid in which degradation and cost is reduced.

Accordingly the present invention provides a process for the preparation of pharmaceutically acceptable quality potassium clavulanic acid by the direct precipitation of clavulanic acid as the potassium salt which has not been pre-purified by the formation of an intermediate amine salt, which the proviso that the subject matter of PCT/GB95/0091 is excluded.

Pharmaceutically acceptable quality potassium clavulanate encompasses material which has a purity of at least 80% as the pure free acid more preferably a purity of at least 82% as the pure free acid and most preferably is purity of at least 83% as the pure free acid.

The above process may be effected by extraction into an organic solvent of the free acid of clavulanic acid resulting from a fermentation process as described in the references above and which may be optionally pre-treated according to the procedures mentioned in the documents above or preferably using techniques hereinafter described. Suitable organic solvents may be used as described in the aforementioned documents.

The extracted clavulanic acid may then be optionally concentrated using techniques conventional in the art or mentioned in documents cited herein or preferably hereinafter described.

The potassium salt of clavulanic acid may then suitably be formed by addition of a suitable potassium salt of an acid such as organic acid.

The thus formed potassium salt may then be conventionally isolated and dried or may be isolated and dried using the methods described herein.

Alternatively, the free acid of clavulanic acid (prepared as above) may be extracted into solvent as described above and optionally concentrated before back extraction of the clavulanic acid into an aqueous medium. The aqueous medium may then be optionally concentrated using conventional techniques or techniques described herein. A suitable form of potassium ions may suitably be added such as in the form of a potassium salt of an acid such as an organic acid. The resulting potassium clavulanate may be precipitated, isolated and dried, for example, by adding an appropriate precipitating solvent such as for example acetone or isopropanol.

The techniques described in the examples are herein incorporated by reference.

It is especially desirable to use organic solvent extracts of clavulanic acid or aqueous extracts of clavulanic acid which are particularly high in titre for example the titre of clavulanic acid in aqueous solution is in the range of 100,000 to 200,000 micrograms per ml more preferably at about 145,000 micrograms per ml.

Preferably the clavulanic acid is obtained from a whole broth fermentation which is preferably filtered by ultra filtration preferably optionally pre-concentrated by reverse osmosis. The aqueous back extract is preferably at a concentration of 10 to 30% more preferably at a concentration of 15 to 25% and most preferably at about 20% w/v.

Preferably a co-solvent may be added to the aqueous concentrate such as isopropanol and any resulting oil is removed by conventional means such as filtration through a celite bed. This is particularly advantageous in allowing the required purity of potassium clavulanate to be formed.

The product is then suitably precipitated by addition of further water miscible solvent such isopropanol optionally admixed with a water immiscible solvent such as ethyl acetate or alternatively by the step-wise addition of the water miscible solvent then the water immiscible solvent.

In the case of isolated of the potassium salt obtained directly from an organic solvent solution, the organic solvent is preferably dried preferably using azeotopic distillation.

Suitable organic solvents include those described above, for example n-butanol, ethyl acetate, n-butyl acetate, and ketones of the general formula R¹ CO.R² where R¹ and R² are independently C₁₋₁₀ alkyl groups, in particular methyl isobutyl ketone. The solution of clavulanic acid may contain impurities, for example high molecular weight impurities such as may be present if the solution has been obtained by a primary isolation process as described above, but preferably has been subjected to a preliminary purification process to remove at least some of the impurities. Suitable preliminary purification processes include filtration, and treatment with absorbent carbon. The solution may also contain small quantities of dissolved or suspended water, but preferably if the solution has been obtained from a primary isolation process it may be subjected to a dewatering process, for example centrifuging to remove droplets of suspended water.

A suitable solution concentration for the solution of clavulanic acid or its labile derivative is around 500 to 20,000 µg/ml (0.0025M to 0.1M), for example around 1,000 - 5,000 µg/ml (i.e. 0.005M to 0.025M), typically around 3,000 ± 1,000 µg/ml (i.e. 0.015 M ± 0.005 M) expressed in terms of clavulanic acid content. Suitable labile derivatives of clavulanic acid include readily-cleaved esters, such as silyl esters. The term "clavulanic acid" as herein after used refers to both free clavulanic acid and such labile derivatives.

Suitable salt forming cations are alkali metal cations and alkaline earth metal cations, in particular potassium. Suitable counter anions include basic anions, such as hydrogen carbonate, carbonate or hydrogen phosphate, and in particular anions of weak organic carboxylic acids, such as those of formula R-CO₂H where R is C₁₋₂₀ alkyl, for example C₁₋₈ alkyl. Suitable carboxylic acids include acetic, propionic and ethyl hexanoic, such as 2-ethyl hexanoic acid.

Suitable salt precursor compounds including these ions are sodium or potassium hydrogen carbonate, potassium hydrogen phosphate or calcium carbonate, and particularly in the case of preparation of potassium clavulanate, potassium 2-ethyl hexanoate. Other suitable salt precursor compounds include ionexchange resins, which may be solid or liquid, and which incorporate a salt-forming cation such as potassium which can form a salt with clavulanic acid.

The clavulanic acid or derivative may be contacted in solution with the salt precursor compound by dissolving or suspending the precursor compound in a solvent, and mixing the two solutions or the solution and suspension. The same organic solvent may be used for the clavulanic acid and the precursor. In the case of potassium 2-ethyl hexanoate as salt precursor compound such a solution in an organic solvent such as methyl isobutyl ketone may suitably be 0.5 to 5.0 M, e.g. 1.0 to 3.0 M, suitably 2.0 ± 0.5M in potassium 2-ethyl hexanoate.

Water may be provided in the contact region in a number of ways as discussed below, and one or more of these ways may be used as alternatives or together. For example the salt precursor compound may itself be dissolved or suspended in water or water containing dissolved organic solvent, and contacted as such with the solution of clavulanic acid. For example the solution of clavulanic acid may contain dissolved or suspended water, e.g. as mentioned above. For example the salt precursor compound may be dissolved or suspended in an organic solvent, e.g. the same solvent as the clavulanic acid is dissolved in, and these solvents may itself include dissolved or suspended water. For example methyl isobutyl ketone may be used as such an organic solvent for the clavulanic acid and the precursor, and may include 0.1 to 7.5% v:v of dissolved water, typically 1 to 3%, e.g. 2.0 ± 0.5%. For example water may be provided by adding water or an aqueous medium such as water containing dissolved organic solvent to the clavulanic acid solution and solution or suspension of the salt precursor in an organic solvent, as they are brought into contact in the contact region.

When dissolved water is present in the organic solvents used in the process of the invention, e.g. as described above, it may subsequently separate out as the aqueous phase by a "salting out" effect as it accumulates the dissolved clavulanic acid salt.

The working conditions, e.g. concentrations of reactants, relative proportions of solutions used, flow rates, contact times etc., of the process are selected such that *inter alia* as much as possible of the clavulanic acid is extracted from the solution in the organic solvent into the aqueous phase as the solution of its salt, and such that a concentrated solution of the salt of clavulanic acid in the aqueous phase is formed. In the case of potassium clavulanate, in a preferred embodiment working conditions are selected so as to produce a concentration of potassium clavulanate in the aqueous phase of ca. 10 to 40 weight % (ca. 0.4 to 1.7 M), for example 20 to 30 weight % (ca. 0.8 to 1.2 M). A solution concentration of potassium clavulanate of this concentration is found to expediate the further processing step, with an optimised yield and improved purity.

In the process, monitoring of the concentration of the clavulanic acid salt such as potassium clavulanate in the separated aqueous phase, for example by density, optically etc. is a suitable way of determining and controlling the other working conditions.

The clavulanic acid and the salt precursor compound should be introduced such that there is an initial stoichometric excess of the salt precursor compound over the clavulanic. For example the precursor may be introduced in a 1 : 1.1 to 1 : 2 molar ratio clavulanic acid : precursor compound, typically 1 : 1.1 to 1 : 1.5, to ensure that there is theoretically sufficient salt precursor compound to combine with all of the clavulanic acid.

The amount of water present in the region in which the clavulanic acid and salt precursor compound are brought into contact should suitably be around the minimum necessary to achieve a desired aqueous phase concentration of the salt, for example, as discussed above for potassium clavulanate.

The process of this invention provides salts of clavulanic acid, for example potassium clavulanate, free of the trace impurities introduced by known purification processes, such as the amines used in the purification process mentioned above. Although salts of clavulanic acid free of such impurities are known on a laboratory scale, the bulk production of such salts, in particular potassium clavulanate, for use in the preparation of pharmaceutical formulations is novel.

Consequently a further aspect of this invention provides a pharmaceutical formulation for the treatment of bacterial infections which comprises a salt of clavulanic acid, e.g. as described above, in particular potassium clavulanate, the formulation being substantially free of organic amines such as t-butylamine, diethylamine, tri-(lower alkyl) amines, methylaniline or NN'-diisopropylethylene diamine (either as free amines or as derivatives or salts thereof).

Suitably the formulation contains less than 0.05%, for example less than 0.005%, preferably less than 0.0005%, desirably less than 0.00005% of organic amines by weight with respect to the weight of the clavulanic acid salt present in the formulation.

The formulation may also comprise one or more antibiotic compounds, suitably β-lactam antibiotics such as penicillins and cephalosporins. Suitable antibiotics include the antibiotics with which clavulanic acid is combined in known antibiotic formulations, for example amoxycillin (e.g. in the form of its trihydrate) and ticarcillin. The formulation may comprise ratios of clavulanic acid salt : antibiotic within the known ranges in which such combinations are used, e.g. 12:1 to 1:1 by weight expressed as in terms of the parent clavulanic acid and antibiotic.

The formulation may also contain other known additives and excipients, e.g. fillers, binders, disintegrants, an effervescent couple, colourants, flavourings, desiccants etc., for example those listed for use with formulations containing potassium clavulanate in GB 2005538. The formulation may also contain materials such as cellulose derivatives, e.g. microcrystalline celluloses such as Avicel (Trade Mark) or Syloid (Trade Mark), silicon dioxide or sucrose together with potassium clavulanate. The formulation may for example comprise a blend of potassium clavulanate with a cellulose derivative, silicon dioxide or sucrose, for example in a 1:1 weight ratio.

The following examples illustrate the present invention:

### Example 1

A crude aqueous solution of clavulanic acid at a titre of about 20,000µg/ml, prepared by either of the following procedures:
(a) RVF filtration of the broth followed by ion exchange chromatography of fermentation broth.
(b) Ultrafiltration of the broth followed by RO concentration of fermentation broth.

The aqueous solution is extracted with ethyl acetate a pHl.5 using sulphuric acid. The rich solvent is chilled and dewatered prior to passing through activated granular carbon column. The purified solvent extract is subsequently distilled azeotropically to give a solution with a clavulanic acid content of about 20,000µg/ml, and a moisture content of 0.2%. Potassium ethyl hexanoate solution is IPA is added to the concentrated solution to achieve precipitation of clavulanate as the potassium salt. The isolated product may require recrystallisation to improve crystal form.

### Example 2

A crude aqueous solution of clavulanic acid at a titre of about 20,000 µg/ml, prepared by either a) or b) as above is extracted with MIBK at pH1.5 sulphuric acid. The rich solvent is chilled and dewatered prior to passing through activated granular carbon column. The purified solvent extract is then back extracted with water at pH5.1 using KEH in a solvent. The whole operation is continuous. The spend solvent is recycled back to the forward extraction and the aqueous extract is recycled until a concentration of 20-25% w/v is reached. The aqueous potassium clavulanate solution is diluted with IPA, treated with powdered carbon then crystallised by the addition of IPA or acetone. A co-solvent may be used at this state.

### Example 3

A sample of clavulanate rich ethyl acetate was prepared as follows:

A crude sample of clavulanic acid was dissolved in water to give a titre approximately 145,000µg/ml, and taken to pH1.5 with sulphuric acid. This was extracted into ethyl acetate, which was subsequently distilled azeotropically to give a solution with a clavulanic acid content of 21.573µg/ml and a moisture content of 0.2%. To 100mls of this was added 7.5mls of a 2N solution of KEH in isopropanol, over a period of 12 minutes, whereat a precipitate was formed. The product, potassium clavulanate was isolated, and the wet cake washed with acetone, then redissolved in 10mls of mixture of methanol and water. 100mls of an isopropanol/mixture was added to this over 20 minutes, whereat white potassium clavulanate precipitated in the form of rods and nucleated rods.
Product weight = 2.03g
Product purity = 83.0% as the pure free acid
Yield from solvent rich solvent = 78%

### Example 4

A crude solution of clavulanic acid was prepared by ultrafiltration of clavulanic acid whole broth followed by RO concentration. An aqueous back extract at a concentration of 20.3% was prepared as in method 2. IPA (100ml) was added to a 100ml of this solution. The resulting oil was removed by filtration through a celite bed. IPA (2,000ml) was slowly added to the filtrate whilst stirring. At the end of the IPA addition, ethyl acetate (300ml) was added and the resultant slurry was chilled and stirred for 1 hour prior to filtration and product drying.
Product weight = 21.0g
Crystallisation stage yield = 86.3%
Product purity = 83.2% as the pure free acid
Product moisture content = 0.3%

## Claims

1. A process for the preparation of pharmaceutically acceptable quality potassium clavulanate by the direct precipitation of clavulanic acid as the potassium salt which has not been pre-purified by the formation of an intermediate amine salt by the reaction of clavulanic acid in solution with potassium ions in solution *characterised:*
by reacting clavulanic acid in solution in an organic solvent with a potassium salt, between 0.1 % v:v and 7.5 % v:v of water being present in the region where the clavulanic acid and potassium salt contact, then isolating the so-formed potassium clavulanate,
with the proviso that a process for the preparation of potassium clavulanate, wherein clavulanic acid in solution in a wholly or partly water-imiscible organic solvent is contacted in a contact region which is a region of high turbulence and/or shear stress, with a compound of potassium with a counter anion in solution or suspension, the counter anion being capable of exchange with clavulanate anion, in the presence of water, such that a solution of potassium clavulanate in an aqueous phase is formed, then the organic solvent and aqueous phases are physically separated during a separation step, followed by a further processing step in which the said salt of clavulanic acid is isolated from solution as a solid is excluded.

2. A process according to claim 1 *characterised* in that 0.2 % v:v to 7.5 % v:v of water is present in the contact region.

3. A process according to claim 2 *characterised* in that 1 % v:v to 3 % v:v of water is present in the contact region.

4. A process according to claim 3 *characterised* in that 2 ± 0.5 % v:v of water is present in the contact region.

5. A process according to any one of claims 1 to 4 *characterised* in that the solution of clavulanic acid in an organic solvent is obtained by extraction of clavulanic acid from an aqueous medium resulting from a fermentation process, followed by concentration and optionally by drying.

6. A process according to any one of claims 1 to 5 *characterised* in that the concentration of clavulanic acid in solution in the organic solvent is 500 microgram / ml to 200,000 microgram / ml.

7. A process according to any one of the preceding claims *characterised* in that a solution of the potassium salt in an organic solvent is added to the solution of clavulanic acid.

8. A process according to claim 7 *characterised* in that the organic solvent in which the potassium salt is dissolved is isopropanol.

9. A process according to any one of the preceding claims *characterised* in that the potassium salt is potassium 2-ethylhexanoate.

10. A process according to claim 7, 8 or 9 *characterised* in that the solution of the potassium salt in an organic solvent is at a concentration 0.5M to 5M.

11. A process according to any one of the preceding claims *characterised* in that the organic solvent in which the clavulanic acid is in solution is selected from n-butanol, ethyl acetate, n-butyl acetate, and ketones of the general formula R¹ CO.R² where R¹ and R² are independently C₁₋₁₀ alkyl groups.

12. A process according to any one of the preceding claims *characterised* in that the potassium clavulanate is allowed or caused to precipitate from the reaction medium.
